# EUROPEAN PATENT SPECIFICATION

(11) **EP 2 514 862 B1**
(45) Date of publication and mention of the grant of the patent: **03.07.2019**
(21) Application number: 12164954.5
(22) Date of filing: 20.04.2012
(51) Int. Cl.: D04B 21/02, A61F 2/00, D04B 21/12

(54) **Medical product and production thereof**
Medizinprodukt und dessen Herstellung
Produit médical et sa production

(30) Priority: 21.04.2011 DE 102011007844
(43) Date of publication of application: 24.10.2012
(73) Proprietor: Aesculap AG, 78532 Tuttlingen/Donau (DE); ITV Denkendorf Produktservice GmbH, 73770 Denkendorf (DE)
(72) Inventor: Odermatt, Erich, 8200 Schaffhausen (CH); Weis, Christine, 8190 Sant Cugat del Vallés (ES); Nieves, Tania, 08225 Terrassa (Barcelona) (ES); Müller, Erhard, 70565 Stuttgart (DE); Schmees, Hans-Gerd, 72827 Wannweil (DE)
(74) Representative: Patentanwälte Ruff, Wilhelm, Beier, Dauster & Partner mbB

(56) References cited:
- EP-A1- 2 229 918
- WO-A1-01/81667
- WO-A1-2011/026987
- US-A- 4 302 953

## Description

The present invention relates to a medical product in the form of a textile fabric and to a corresponding production process.

Self-retaining implants are generally implants that do not need additional fixing aids such as, for example, suture materials, adhesives, clips or the like for retention/anchoring in the body of a human or animal patient. Self-retaining implants have self-anchorable sub-structures in the form of barbs, pile threads, thread loops and the like for example.

Implants having such sub-structures that at least augment anchoring in the body are already known from the following documents for example:
DE 19 912 648 A1 describes a sheetlike implant for use in surgery comprising a flexible fabric, wherein one side of the fabric may include textured yarns, floats and/or velour loops to improve the cellular ingrowth behaviour.

DE 10 2007 063 214 A1 relates to a hernia implant in the form of a first textile fabric, which is coated uniformly and sealingly, and a second textile fabric which is connected to the first fabric in a conjoint connecting plane and which is free of any coating. There may be places on the implant for body cells to get behind, for example textured yarns, floats, velour loops and/or tufts.

WO 2009/071998 A2 discloses an implant for preventing or treating stomal hernias which may have fastening means such as for example loops and barbs on one side for fastening to the abdominal wall.

WO 01/81667 A1 relates to a prosthetic knit for medical or surgical use wherein a ply of monofil threads on one side of the knit has protruding picot hairs.

Polymeric meshes with anchoring hooks useful particularly for the surgical management of laparoceles form part of the subject matter of printed publication DE 20 2004 017 304 U1.

A wound closure device with barbs is known from US 2002/0077661 A1. EP 2 229 918 A1 reveals a self-retaining implant with barbs. This implant is likewise particularly suitable for hernia management.

A prosthetic fabric comprising an arrangement of yarns that define at least first and second opposite faces is known from WO 2011/026987 A1. The fabric comprises on at least the first face one or more barbs that protrude outwards relative to the first face, wherein the fabric is covered, at least partly, on the second face with a layer made of a bioresorbable material and wherein the outer surface of the layer is impenetrable by the barbs, wherein the barbs are, in addition, covered with a coating made of a water-soluble material.

A right-right warp knitting is known from US 4,302,953.

Difficulties can arise with these kinds of implants where the sub-structures provided for retention or self-retention are not firmly integrated in the implant structure. In these cases, there is a risk that the sub-structures will become dislodged out of the implant under tensile stress, directly impairing the structural integrity and hence the (self-)retaining properties of the implant.

It is an object of the present invention to provide a medical product having (self-)retaining properties which evades prior art disadvantages and more particularly provides secure retention/anchoring even under the influence of tensile-load forces as may typically arise in the body of a human or animal patient.

This object is achieved according to a first aspect of the invention by a medical product according to independent claim 1. This medical product comprises at least one textile fabric having a textile ground structure and thread structures which protrude from the textile ground structure, wherein at least some of the thread structures are interlooped with the textile ground structure, i.e. at least some of the thread structures are tied into the textile ground structure in the form of at least one interloop.

In other words, the thread structures - at least some of them - are at one end interlooped with the textile ground structure, while their free ends protrude from the textile ground structure.

The expression "at least one textile fabric" shall for the purposes of the present invention be understood as meaning in general one textile fabric or a multiplicity of textile fabrics, especially two, three or more textile fabrics.

According to the invention the medical product is in the form of a textile fabric having a textile ground structure and thread structures which protrude from the textile ground structure with at least some of the thread structures being interlooped with the textile ground structure, i.e. at least some of the thread structures being tied into the textile ground structure in the form of at least one interloop.

The medical product according to the present invention is advantageous because the thread structures which are interlooped with the textile ground structure cannot be pulled out of the textile ground structure on exposure to tensile loads typically arising in the body of a patient. As a result, the thread structures, which in functional respects can be conceived of as retaining or anchoring structures, are able to contribute to a secure/reliable retention, especially self-retention, of the product in human or animal tissue. There is accordingly no risk of the thread structures becoming lost under tensile stress and thereby possibly causing an undesired dislocation of the product in the body of a patient.

The interlooping of the thread structures with the textile ground structure preferably also has the effect that the stiffness of the medical product is increased, which is advantageous especially with regard to the unfurlability of the medical product, for example after delivery from a suitable delivering instrument such as a trocar in particular.

The expression "in the form of at least one interloop" shall for the purposes of the present invention be understood as meaning in general "in the form of one interloop and/or in the form of a multiplicity of interloops, for example in the form of two, three and/or four interloops".

In one preferred embodiment, all thread structures are interlooped with the textile ground structure, i.e. tied into the textile ground structure in the form of at least one interloop.

According to the invention, at least some of the thread structures are tied pairwise into the textile ground structure in the form of at least one interloop.

The at least one interloop is configured as pillar, tricot, atlas, cord, filet and/or velvet stitch.

In a further embodiment, threads, preferably all threads, of the at least one textile fabric, especially of the textile ground structure and/or the thread structures, are free from anchoring structures, especially sticking-out anchoring structures, such as barbs for example.

More particularly, threads, preferably all threads, of the at least one textile fabric, especially of the textile ground structure and/or the thread structures, have no incisions on the thread surface, especially no anchoring structures configured as incisions on the thread surface. This has the advantage with regard to the thread structures that the thread diameter has no incision-caused taperings which could possibly impair the mechanical stability and especially the (self-)retaining properties of the medical product.

The thread structures are preferably threads, especially severed, preferably cut, cut-off or cut-through, threads.

Preferably, the thread structures are configured as tuft/pile threads, especially as velour threads, velvet threads, plush threads or combinations thereof, or in the manner of tuft/pile threads, especially in the manner of velour threads, velvet threads, plush threads or combinations thereof.

The thread structures in a further embodiment have a cut area at their free ends.

The thread structures may be configured cylindrically or essentially cylindrically at their free ends, in which case the cylindrical basal areas at the free ends are preferably each configured as cut area.

The thread structures in a further embodiment project from the textile ground structure perpendicularly or essentially perpendicularly. The expression "essentially perpendicularly" is here to be understood as meaning that the thread structures can optionally project away from the textile ground structure at an angle below 90 °, especially at an angle between 45 and 90 °, preferably at an angle between 70 and 90 ° and more preferably at an angle between 80 and 90 °. An angle < 90 ° may be advantageous with regard to lower tissue irritation when fixing the product of the present invention in the body of a patient.

In the case of thread structures projecting perpendicularly from the textile ground structure, the extent of tissue irritation can be controlled with particular advantage through the length with which the thread structures project from the textile ground structure.

The thread structures may further have a straight-line or essentially straight-line body and have a head at each of their free ends. The head is preferably wider than the body of the thread structures. For example, the head may be widened relative to the body in the manner of a mushroom head. A head that is broader than the body may be advantageous in tissue fixation.

The thread structures in a particularly preferred embodiment and more particularly on at least one side of the at least one textile fabric are severed, preferably cut-through, spacer or connecting threads of a spacer fabric, especially of a knitted spacer fabric, in particular a loop-formingly knitted spacer fabric, preferably of a spacer fabric configured as purl fabric or rib fabric, preferably rib fabric.

A purl fabric for the purposes of the present invention is generally a textile fabric, especially a spacer fabric, where the technical back and the technical face of the fabric, especially the outer technical back and the outer technical face of the fabric, each show reverse interloops.

A rib fabric for the purposes of the present invention is generally a textile fabric, especially a spacer fabric, where the technical back and the technical face of the fabric, especially the outer technical back and the outer technical face of the fabric, each show face interloops.

A plain-knit fabric for the purposes of the present invention is generally a textile fabric, especially a spacer fabric, whose technical face, especially exterior technical face, shows face interloops and whose technical back, especially exterior technical back, shows reverse interloops.

Each interloop or interlooped stitch of an interlooped fabric such as a formed-loop knit for example generally consists of a head, two legs and two feet. Where the legs transition into the feet there are two points of contact with the preceding interloop which are known as intermeshing points. When the heads and feet of the interloops point up and the legs correspondingly down at these intermeshing points, reverse interloops and the technical back of an interlooped fabric are concerned. If, by contrast, the feet are down and the legs up, face interloops and the technical face of an interlooped fabric are concerned. With regard to the definition of reverse and face interloops and of the technical back and the technical face, reference may also be made here to standard works in textile technology.

A particular advantage of a spacer fabric configured as rib fabric is that the binding of the spacer or connecting threads in the form of at least one interloop in the ground structure is freely chooseable.

In one particularly preferred embodiment, the at least one textile fabric includes at least two thread plies, especially two or three thread plies, preferably just two thread plies.

In an advanced embodiment, the at least one textile fabric includes two thread plies, of which one thread ply forms the textile ground structure and the other thread ply forms the thread structures.

In alternative embodiments, the at least one textile fabric includes three thread plies, of which two thread plies form the textile ground structure and the third thread ply forms the thread structures.

The thread structures are preferably only configured on one side (one-sidedly), especially just on one surface side, of the textile ground structure. Preferably, in this embodiment, additional thread structures are formed on an opposite side, especially on an opposite surface side, of the textile ground structure and preferably likewise protrude from the textile ground structure. The expression "additional thread structures", hereinafter also called "additionally present thread structures" or "additionally provided thread structures", is to be understood as meaning thread structures which, in addition to the thread structures provided according to the present invention, may protrude from what is generally an opposite side of the textile ground structure.

The thread structures provided according to the invention and the optionally additionally present thread structures may be configured the same or differently. More particularly, the optionally additionally provided thread structures may also be interlooped with the textile ground structure. However, there can be differences, for example with regard to the thread material, the thread diameter, the thread length, especially the tuft or pile length, the thread linear density, the thread structure and the like. Further differences may reside in a different pattern, a different arrangement and/or in a different density with which the thread structures and the optionally additionally present thread structures can be configured on the surface of the textile ground structure.

The additional thread structures may be thread loops, more particularly selected from the group consisting of pile loops, velour loops, velvet loops, terry loops, plush loops, floats and combinations thereof, and/or be tuft/pile threads, more particularly selected from the group consisting of velour threads, velvet threads, plush threads, terry threads and combinations thereof.

In preferred embodiments, the at least one textile fabric has thread structures, especially in the form of severed spacer or connecting threads of a spacer fabric, on one side and thread loops, especially in the form of velour and/or terry loops, on an opposite side.

In alternative preferred embodiments, the at least one textile fabric has the thread structures, especially in the form of severed spacer or connecting threads of a spacer fabric, on one side and sticking-out threads in the form of tuft or pile threads on an opposite side.

The thread structures and/or the optionally additionally present thread structures may be monofil, pseudomonofil and/or multifil, especially braided or twisted. However, it is particularly preferable according to the present invention for the thread structures and/or the optionally additionally present thread structures to have a monofil, pseudomonofil and/or monofilament-like configuration. As a result, the thread structures and/or the optionally additionally present thread structures have a higher flexural stiffness, which is advantageous especially with regard to the retention of the product of the present invention in the human or animal body.

The thread structures and/or the optionally additionally present thread structures may have a diameter between 50 and 300 µm, especially 80 and 250 µm and preferably 80 and 160 µm.

The thread structures and/or the optionally additionally present thread structures may further be configured on the textile ground structure in a density, especially tuft density, between 10 and 330 per cm², especially 19 and 225 per cm² and preferably 20 and 50 per cm².

The thread structures and/or the optionally additionally present thread structures in a further embodiment have a length, especially a tuft length, of 0.1 to 5 mm, especially of 0.5 to 4 mm and preferably of 0.5 to 2.5 mm.

The medical product, especially the at least one textile fabric, preferably the textile ground structure, the thread structures and/or the optionally additionally present thread structures, may be configured to be absorbable, partially absorbable and/or non-absorbable.

It is preferable for the medical product and especially for the at least one textile fabric to have at least one absorbable fraction. This makes it possible to reduce the proportion of material remaining permanently in the body of a patient, resulting in effect in a reduced input of exogenous material into the body of a patient.

In a further embodiment, the medical product, especially the at least one textile fabric, preferably the textile ground structure, the thread structures and/or the optionally additionally present thread structures, is formed of an absorbable material, especially of an absorbable polymer. The polymer may be a synthetic/manufactured polymer and/or a so-called biopolymer, i.e. a naturally occurring polymer such as for example a protein and/or a polysaccharide. It is preferable for the absorbable polymer to be a polyester, especially a polyhydroxyalkanoate.

In an advanced embodiment, the absorbable polymer is selected from the group consisting of polylactide, polyglycolide, poly-ε-caprolactone, poly-para-dioxanone, polytrimethylene carbonate, polyhydroxybutyrate, poly-3-hydroxybutyrate, poly-4-hydroxybutyrate, polyhydroxyvalerate, copolymers, for example terpolymers, thereof and combinations thereof. Copolymers for the purposes of the present invention are polymers composed of two or more, for example three, different monomeric units. Copolymers may be random copolymers or block copolymers.

Alternatively or in combination to the preceding embodiments, the absorbable polymer may be selected from the group consisting of collagen, gelatin, elastin, reticulin, laminin, fibronectin, silk, especially spider's silk, albumin, fibrin, fibrinogen, starch, amylose, amylopectin, dextran, chitosan, cellulose, alkylcelluloses, especially methylcellulose, carboxyalkylcelluloses, especially carboxymethylcellulose, hyaluronic acid, heparin, heparan sulphate, chondroitin sulphate, dermatan sulphate, keratan sulphate, salts thereof and combinations thereof.

One example of a preferred absorbable copolymer is a copolymer comprising glycolide and lactide, especially in a ratio of 95 wt%:5 wt% to 5 wt%:95 wt% and preferably of 90 wt%:10 wt% to 80 wt%:20 wt%. A further example of a preferred copolymer is a copolymer comprising L-lactide and D,L-lactide, especially in a ratio of 95 wt%:5 wt% to 5 wt%:95 wt%, preferably 80 wt%:20 wt% to 20 wt%:80 wt% and more preferably 70 wt%:30 wt% to 30 wt%:70 wt%. A preferred terpolymer is a terpolymer, especially block terpolymer, comprising glycolide, trimethylene carbonate and ε-caprolactone. A terpolymer of this type is commercially available under the designation Monosyn®.

In a further embodiment, the medical product, especially the at least one textile fabric, preferably the textile ground structure, the thread structures and/or the optionally additionally present thread structures, are formed of a non-absorbable material, especially of a non-absorbable polymer, preferably selected from the group consisting of polyolefins, polyesters, polyamides, polyurethanes, copolymers, for example terpolymers, thereof and combinations thereof.

The non-absorbable polymer in an advanced embodiment is selected from the group consisting of polypropylene, polyethylene, high density polyethylene (HDPE), low density polyethylene (LDPE), high molecular weight polyethylene (HMWPE), ultrahigh molecular weight polyethylene (UHMWPE), polyvinylidene dichloride, polyvinylidene difluoride, polytetrafluoroethylene, polyhexafluoropropylene, polytetrafluoropropylene, polyethylene terephthalate, polypropylene terephthalate, polybutylene terephthalate, polyetheretherketone (PEEK), nylon, nylon-6, nylon-6,6, nylon-6,12, polyurethane, copolymers, for example terpolymers, thereof and combinations thereof.

In a particularly preferred embodiment, the thread structures and/or the optionally additionally present thread structures are configured to be absorbable, while the textile ground structure is configured to be partially absorbable or non-absorbable, preferably non-absorbable.

An advantage of thread structures configured to be absorbable is that absorption events in vivo are generally associated with inflammatory reactions which stimulate the formation of new connective tissue. This usually means in the present case a stimulation of connective tissue in the region of the thread structures which enables improved retention in the body of a patient to be achieved for the product according to the present invention.

It may further be provided according to the present invention for the at least one textile fabric, especially the textile ground structure, the thread structures and/or the optionally additionally present thread structures, to be formed of a mixture of materials described above, especially polymers.
In one advantageous embodiment, the thread structures and/or the optionally additionally present thread structures may be formed of, or be coated with, a material, especially polymer, which is swellable in fluids, especially bodily fluids such as for example tissue fluids and/or blood, and/or is tissue adhesive. The material is preferably present as hydrogel. Suitable materials may be selected for example from the group consisting of polyvinyl alcohol, polyvinylpyrrolidone, polypropylene glycol, polypropylene oxide, polytetramethylene oxide, proteins, polysaccharides, especially mucopolysaccharides, copolymers, for example terpolymers, thereof, salts thereof and combinations thereof.

Proteins may be fibre-shaped, especially extracellular, proteins and/or globular proteins. Preferred proteins are selected from the group consisting of collagen, gelatin, elastin, reticulin, laminin, fibronectin, albumin, fibrin, fibrinogen, salts thereof and combinations thereof.

Suitable polysaccharides may be selected from the group consisting of starch, amylose, amylopectin, dextran, chitosan, cellulose, alkylcelluloses, especially methylcellulose, carboxyalkylcelluloses, especially carboxymethylcellulose, hyaluronic acid, heparin, heparan sulphate, chondroitin sulphate, dermatan sulphate, keratan sulphate, salts thereof and combinations thereof.

The textile ground structure may further be formed of a different material/polymer or a different material/polymer mixture than the thread structures and/or the optionally additionally present thread structures. With regard to possible materials/polymers, full reference is made to the description hereinabove.

However, it is similarly preferable according to the present invention for all sub-structures of the at least one textile fabric, especially the textile ground structure, the thread structures and the optionally additionally present thread structures, to be formed of the same polymer/material or the same material/polymer mixture. With regard to possible materials/polymers, full reference is likewise made to the description hereinabove.

The thread structures and/or the optionally additionally present thread structures may have a core-sheath construction in a further embodiment. For example, the core may be formed of a non-absorbable material, especially non-absorbable polymer, and the sheath of an absorbable material, especially an absorbable polymer. The choice of material for core and sheath can be used to specifically adjust/control the absorbability of the thread structures and/or of the optionally additionally present thread structures in particular.

The thread structures and/or the optionally additionally present thread structures may in particular be bicomponent threads. The expression "bicomponent threads" is to be understood for the purposes of the present invention as meaning threads which are formed of two different materials, especially two different polymers.

In a further advantageous embodiment, sub-structures of the at least one textile fabric, especially the textile ground structure, the thread structures and/or the optionally additionally present thread structures, may absorb at different rates, i.e. have different absorption times. It may be provided for example that the thread structures and/or the optionally additionally present thread structures have a shorter absorption time than the textile ground structure.

In a further embodiment, the thread structures and/or the optionally additionally present thread structures are arranged in the form of a pattern, for example in the form of a circular, oval-shaped, triangular, quadrangular, rectangular, square, pentangular and/or hexangular pattern, on the surface of the textile ground structure.

In addition, the thread structures and/or the optionally additionally present thread structures may be arranged on the surface of the textile ground structure in an arrangement selected from the group consisting of row-shaped arrangement, staggered arrangement, overlapping arrangement, spiral- or helical-shaped arrangement, serpentine-shaped arrangement, meander-shaped arrangement, adventitious or random arrangement and combinations thereof.

The textile ground structure typically has a textile structure based on threads, preferably polymer threads. With regard to the material/polymer of which the threads may be formed, reference is made to the materials/polymers mentioned in the present description.

The textile ground structure is preferably configured as interlooped fabric, especially as formed-loop knit or drawn-loop knit, preferably as formed-loop knit, especially as warp knit.

The textile ground structure is further preferably configured to be porous.

More particularly, the textile ground structure may have interloops, especially pores, having a clear size/diameter of 100 to 10 000 µm, especially of 250 to 8000 µm and preferably of 500 to 5000 µm.

The textile ground structure according to the invention has interloops configured as pillar, tricot, cloth, atlas, filet and/or velvet stitches.

It is particularly preferable for the textile ground structure to be configured as a mesh, preferably as a loop-formingly knitted mesh and especially as a warp-knitted mesh.

The at least one textile fabric in a further embodiment may have a basis weight between 40 and 200 g/m², especially 50 and 100 g/m².

It is preferable for the at least one textile fabric to have a basis weight between 25 and 100 g/m² and especially 25 and 75 g/m² after absorption of an absorbable fraction.

In a further embodiment, the thread structures protrude from the textile ground structure on one side of the textile fabric only, while the opposite side has a coating, especially a uniform and preferably sealing coating. The coating may be formed for example of collagen, gelatin, polyvinyl alcohol, salts thereof or the like. It is preferable for the coating to have anti-adhesive properties.

In a further suitable embodiment, the medical product comprises two textile fabrics, while at least one of the two textile fabrics, especially only one of the two textile fabrics, has a textile ground structure with thread structures which protrude from the textile ground structure, while at least some of the thread structures are interlooped with the textile ground structure.

In preferred embodiments, both the textile fabrics each have a textile ground structure and thread structures which protrude from the textile ground structure, while at least some of the thread structures are interlooped with the textile ground structure.

According to the present invention, the two textile fabrics, especially their textile ground structures, thread structures and/or their optionally additionally present thread structures, may be configured to be the same or different. A differing configuration of the textile fabrics may be based for example on different textile structures, intermeshings, thread materials, thread diameters, thread linear densities, thread structures, interloop/pore shapes, interloop/pore sizes, basis weights, absorbability/nonabsorbability, absorption times and/or other properties.

According to the present invention, the textile ground structure of one textile fabric may be present for example as interlooped fabric, especially as formed-loop knit or drawn-loop knit, while the textile ground structure of the other textile fabric is a non-woven structure, especially a fibrous nonwoven web or a laid scrim.

However, it is particularly preferable according to the present invention for the two textile fabrics to be configured the same, especially their textile ground structures each being configured as interlooped fabric, preferably as formed-loop knit, and especially as a loop-formingly knitted mesh.

The two textile fabrics in an advanced embodiment are in connection with each other. More particularly, the two textile fabrics may be interlinked.

In preferred embodiments, the two textile fabrics are arranged/laid one above the other. It is particularly preferable for at least some of the thread structures, especially all the thread structures, of the bottom textile fabric to project through interloops, especially pores, in the top textile fabric. The bottom textile fabric in this arrangement and more particularly thread structures protruding from its ground structure may be bonded, more particularly adhered, thermofixed and/or fused, more particularly ultrasonically fused, to the textile ground structure of the top textile fabric. It is preferable for the bottom textile fabric to be configured to be absorbable and for the top textile fabric to be configured to be non-absorbable. The top textile fabric may be formed of polypropylene for example. The embodiments described in this section have the advantage that they make it possible to increase the thread structures on one side of the product which are provided for retaining, especially self-retaining, the product in the body of a patient. The other side, by contrast, may for example have a coating, preferably a smooth and preferably sealing, and more particularly anti-adhesive, coating and/or additional thread structures within the meaning of the present invention.

With regard to further features and advantages relating to the embodiments whereby the medical product may comprise two textile fabrics, full reference is made to the invention description hereinabove and hereinbelow.

The at least one textile fabric may in principle be configured as double-backed fabric, single-faced fabric or double-faced fabric. It is preferable for the at least one textile fabric to be embodied as single-faced fabric, preferably as interlooped single-faced fabric and more particularly as loop-formingly plain-knitted fabric.

In a further embodiment, the medical product, especially the at least one textile fabric and preferably the textile ground structure, the thread structures and/or the optionally additionally present thread structures, is provided with additives, especially active agents such as for example active medical/pharmaceutical agents, active biological agents and/or other active agents. The active agents may be selected for example from the group consisting of active antimicrobial, especially antibiotic, agents such as for example silver, active disinfecting agents, active antiinflammatory agents, active analgesic agents, active agents that promote cellular growth, active agents that stimulate cellular differentiation, active agents that promote cellular migration, active agents that promote cellular adhesion, salts thereof and combinations thereof.

The medical product is preferably configured as surgical implant, especially as self-retaining implant. Self-retention on the part of an implant according to the present invention is obtainable for example by the implant being laid onto the tissue surfaces to be treated/managed and pressed down onto the tissue surfaces which forces the thread structures into the tissue and ensures a generally sufficient degree of retention of the implant in the body of a patient.

The medical product is preferably configured as implant for treatment/management of hernias, thoracal defects, prolapses, urinary incontinence and/or dyspareunia in man and/or animal.

It is particularly preferable for the medical product to be a surgical mesh, especially a self-retaining mesh.

The medical product in an advanced embodiment is selected from the group consisting of hernia mesh, prolapse mesh, incontinence mesh, dyspareunia mesh, stoma mesh and thorax mesh.

Further, it is disclosed a medical product comprising a spacer fabric with at least two, especially two, opposite textile ground structures spaced apart from each other via spacer threads (connecting threads), wherein at least some of the spacer threads are interlooped with at least one of the two textile ground structures, especially with both textile ground structures, i.e. at least some of the spacer threads being tied in the form of at least one interloop in at least one of the two textile ground structures and especially into both textile ground structures.

Preferably, all spacer threads are interlooped with at least one of the two textile ground structures and especially with both textile ground structures, i.e. tied in the form of at least one interloop in at least one of the two textile ground structures and especially into both textile ground structures.

The spacer threads may be merely interlooped with one of the two textile ground structures, and are not interlooped with the other textile ground structure and more particularly are connected to the other textile ground structure in some other way. For example, the spacer threads can be adhered, thermofixed and/or fused to the other textile ground structure.

Further, threads, preferably all threads, of the spacer fabric, especially of the textile ground structures, and/or the spacer threads, may be free from anchoring structures, especially sticking-out anchoring structures, for example barbs.

More particularly, threads, preferably all threads, of the spacer fabric, especially of the textile ground structures, and/or the spacer threads, have no incisions on the thread surface, especially no anchoring structures configured as incisions on the thread surface.

The spacer threads may in principle be configured as monofil, pseudomonofil and/or multifil, especially braided or twisted. However, it is particularly preferable according to the present invention for the spacer threads to be configured as monofil, pseudomonofil and/or monofilament-like.

Further, the two textile ground structures may have projecting thread structures on that side which faces away from the spacer threads. In a possible alternative, only one of the two textile ground structures has projecting thread structures on that side which faces away from the spacer threads. The thread structures preferably serve to retain, especially self-retain, the medical product or spacer fabric. The thread structures may be configured as thread loops, more particularly selected from the group consisting of pile loops, velour loops, velvet loops, terry loops, plush loops, floats and combinations thereof, and/or as tuft/pile threads, more particularly selected from the group consisting of velour threads, velvet threads, plush threads, terry threads and combinations thereof.

Further, the spacer threads may be interposed with thread loops, more particularly selected from the group consisting of velour loops, velvet loops, terry loops, plush loops, floats and combinations thereof. More particularly, one thread loop may be configured between two spacer threads at a time.

The two textile ground structures of the spacer fabric may in principle be configured the same or differently. When the textile ground structures are configured differently, the differing configuration may be based for example on different intermeshings, thread materials, thread diameters, thread linear densities, thread structures, textile structures, interloop/pore shapes, interloop/pore sizes, basis weights, absorbability/nonabsorbability, absorption times and/or other properties.

In principle, the spacer fabric, especially at least one of the two textile ground structures and preferably both textile ground structures, and/or the spacer threads, may be configured to be absorbable, partially absorbable and/or non-absorbable.

Further, the spacer threads may be configured to be absorbable.

Further, at least one of the two textile ground structures, especially only one of the two textile ground structures, may be configured to be absorbable.

It may further be preferable for one of the two textile ground structures to be configured to be absorbable and for the other textile ground structure to be configured to be non-absorbable.

Further, the spacer threads and one of the two textile ground structures may be configured to be absorbable, while the second textile ground structure is preferably configured to be non-absorbable.

Further, the medical product or spacer fabric, especially at least one of the two textile ground structures and preferably both textile ground structures, and/or the spacer threads, may be formed of an absorbable material, especially an absorbable polymer, for example a synthetic/manufactured polymer and/or a biopolymer, preferably a polyhydroxyalkanoate.

The absorbable polymer may be selected from the group consisting of polylactide, polyglycolide, poly-ε-caprolactone, poly-para-dioxanone, polytrimethylene carbonate, polyhydroxybutyrate, poly-3-hydroxybutyrate, poly-4-hydroxybutyrate, polyhydroxyvalerate, copolymers, for example terpolymers, thereof and combinations thereof. Alternatively or in combination to the preceding embodiments, the absorbable polymer may be selected from the group consisting of collagen, gelatin, elastin, reticulin, laminin, fibronectin, silk, especially spider's silk, albumin, fibrin, fibrinogen, starch, amylose, amylopectin, dextran, chitosan, cellulose, alkylcelluloses, especially methylcellulose, carboxyalkylcelluloses, especially carboxymethylcellulose, hyaluronic acid, heparin, heparan sulphate, chondroitin sulphate, dermatan sulphate, keratan sulphate, salts thereof and combinations thereof.

Further, the medical product or the spacer fabric, in particular at least one of the two textile ground structures and preferably both textile ground structures, and/or the spacer threads, may be formed of a non-absorbable material, especially of a non-absorbable polymer, preferably selected from the group consisting of polyolefins, polyesters, polyamides, polyurethanes, polyetheretherketones, copolymers, for example terpolymers, thereof and combinations thereof.

The non-absorbable polymer may be selected from the group consisting of polypropylene, polyethylene, high density polyethylene (HDPE), low density polyethylene (LDPE), high molecular weight polyethylene (HMWPE), ultrahigh molecular weight polyethylene (UHMWPE), polyvinylidene dichloride, polyvinylidene difluoride, polytetrafluoroethylene, polyhexafluoropropylene, polytetrafluoropropylene, polyethylene terephthalate, polypropylene terephthalate, polybutylene terephthalate, polyetheretherketone (PEEK), nylon, nylon-6, nylon-6,6, nylon-6,12, polyurethane, copolymers, for example terpolymers, thereof and combinations thereof.

Further, at least one of the two textile ground structures, especially both textile ground structures, may be configured as interlooped fabric such as for example formed-loop knit or drawn-loop knit or as non-woven structure such as for example fibrous nonwoven web or laid scrim.

It is preferable for at least one of the two textile ground structures, especially both textile ground structures, to be embodied as interlooped fabric, preferably as formed-loop knit and especially as warp knit.

It is particularly preferable for at least one of the two textile ground structures, especially both textile ground structures, to be present as mesh, preferably as loop-formingly knitted mesh and especially as warp-knitted mesh.

The spacer threads may be interlooped with a loop-formingly knitted and preferably mesh-configured textile ground structure and connected to an opposite, preferably fibrous nonwoven web type, textile ground structure in a manner other than interlooping, especially by adhering, thermofixing and/or fusing.

Further, the spacer fabric may comprise more than two, for example three, opposite textile ground structures. The ground structures are preferably spaced apart from each other via spacer threads which are at least partly interlooped with the ground structures. With regard to further features and advantages concerning the spacer fabric, especially the textile ground structures and/or the spacer threads, the description hereinabove is referenced in full.

The spacer fabric itself may in principle be embodied as double-backed fabric, single-faced fabric or double-faced fabric. The spacer fabric is preferably embodied as double-faced fabric, especially as interlooped double-faced fabric and preferably as loop-formingly knitted double-faced or rib fabric.

The medical product or the spacer fabric, especially at least one of the two textile ground structures, preferably both textile ground structures, and/or the spacer threads, may further include additives, especially active agents. With regard to the possible additives, especially active agents, the description hereinabove is referenced in full.

The medical product is preferably a surgical implant, especially a self-retaining implant.

With regard to further features and advantages of the medical product or spacer fabric, especially of the two textile ground structures, and/or the spacer threads, the observations made in connection with the first aspect of the invention are referenced in full.

A second aspect of the present invention provides a process for producing two medical products, wherein spacer threads of a spacer fabric which space apart two mutually opposite textile ground structures from each other are severed, preferably cut through, to obtain two textile fabrics having a textile ground structure and thread structures protruding from the textile ground structure. The two textile fabrics each may serve as a medical product having the features recited in independent claim 1.

The two textile ground structures may be reconnected to each other, if desired. Any suitable physical and/or chemical method may be contemplated for this in principle.

In one advantageous embodiment, the two textile fabrics obtained may be arranged/laid on top of each other for example such that at least some of the thread structures, especially all thread structures of the bottom textile fabric project through interloops, especially pores, in the top textile fabric. To connect the two textile fabrics together, the bottom textile fabric and more particularly thread structures protruding from its ground structure, may be adhered, thermofixed and/or fused, especially ultrasonically fused, to the ground structure of the top textile fabric.

In one preferred embodiment, the spacer threads are interlooped with at least one of the two textile ground structures, preferably with both textile ground structures, to produce the spacer fabric.

The spacer threads are tied into both textile ground structures, in the form of at least one interloop.

Preferably, the spacer fabric is produced by formed-loop knitting.

The spacer fabric in an advanced embodiment is produced using a formed-loop knitting machine and especially a three-dimensional formed-loop knitting machine. The formed-loop knitting machine preferably has at least two and especially exactly two formed-loop knitting needle beds. Each formed-loop knitting needle bed typically has formed-loop knitting needles and also a knock-over comb bar. The distance between the knock-over comb bar and the formed-loop knitting needle bed can be used to determine the distance between the two textile ground structures in the final spacer fabric.

By using a three-dimensional formed-loop knitting machine, the length and/or density of the spacer threads and hence of the thread structures resulting therefrom after severing the spacer or connecting threads can be widely varied for example. Using a three-dimensional formed-loop knitting machine further allows the production of spacer fabrics having two differently configured textile ground structures. For instance, a three-dimensional formed-loop knitting machine enables one-step production of two textile ground structures which are mutually spaced apart via spacer threads and which differ especially in respect of the textile type of intermeshing or lapping, the thread material, the thread diameter, the thread linear density, the thread structure, the interloop/pore shape (for example hexagonal structures, diamond structures, lattice structures and the like), the interloop/pore size, the basis weight, the absorbability/nonabsorbability, the absorption time and/or other properties.

Depending on the materials used, the textile fabrics obtained after severing the spacer or connecting threads may, as already mentioned, also be reconnected again physically and/or chemically.

Furthermore, textile ground structures having large repeats can be produced using a three-dimensional formed-loop knitting machine.

In principle, the spacer fabric can be produced as double-faced fabric, single-faced fabric or double-backed fabric. The spacer fabric is preferably produced as double-faced fabric. Producing the spacer fabric as double-faced fabric has the particular advantage that the threading of the spacer threads is freely chooseable. It is possible, for example, to use six fully threaded guide bars and six empty guide bars per repeat of the spacer fabric or of the two textile ground structures. Alternatively, two fully threaded guide bars and two empty guide bars can be used per repeat. Alternatively, one fully threaded guide bar and one empty guide bar can be used per repeat. It is otherwise likewise possible according to the present invention for fully threaded guide bars, for example six fully threaded guide bars, to be used exclusively per repeat.

The spacer threads may in principle be severed during the production of the spacer fabric or thereafter.

Preferably, the spacer threads are severed at or in a formed-loop knitting machine. Useful cutting instruments for this can more particularly be a structural component of the formed-loop knitting machine for example. This enables precise and more particularly controlled severing of spacer threads.

It is particularly preferable for the spacer threads to be severed immediately after the interlooping (interloop-forming process), preferably at or in a three-dimensional formed-loop knitting machine.

The spacer threads in an advanced embodiment are severed, preferably cut through, mechanically, thermally, for example using a hot wire, using ultrasound or using a laser, preferably on a formed-loop knitting machine. When the spacer threads are severed thermally, especially with a hot wire, the thread structures resulting therefrom generally have head-shaped, more particularly mushroom head-shaped, widenings at their free ends.

In a further embodiment, thread loops are produced on at least one of the two textile ground structures and especially on both textile ground structures on that side which faces away from the spacer threads or thread structures. The thread loops may be formed during or after the production of the spacer fabric. Severing the spacer threads provides with particular advantage one textile fabric or two textile fabrics which each have one side with thread structures protruding from the textile ground structure in the form of severed spacer threads and an opposite side with thread loops. The thread loops may be produced via alternating thread insertions using needles and/or pins. The thread loops may be further selected from the group consisting of pile loops, velour loops, velvet loops, terry loops, plush loops, floats and combinations thereof.

In a further embodiment, the thread loops are severed. This is a particularly advantageous way of obtaining two textile fabrics which have thread structures in the form of severed spacer threads on one side and severed/opened thread loops or a cut pile (cut pile threads) on an opposite side.

In a further suitable embodiment, thread loops can be produced between the spacer threads during the production of the spacer fabric. More particularly, one or optionally more than one thread loop can be produced between any two adjacent spacer threads. Such thread loops are readily obtainable during the production process of the spacer fabric using a formed-loop knitting machine and especially a three-dimensional formed-loop knitting machine.

The advantage of the process described in the context of the third aspect of the present invention is chiefly that one production step provides two medical products each in the form of a textile fabric having thread structures protruding from its textile ground structure, especially in the manner of a cut pile, without any need for subsequent processing steps such as the severing of thread loops for example.

By contrast, conventional processes for producing products comprising a cut loop pile initially involve producing thread loops out of a textile ground structure which have to be severed in a subsequent step to form a cut loop pile.

A further advantage of the process according to the present invention is that the two textile fabrics obtained can be joined together to form a novel implant, especially a composite implant. In this regard, full reference is made to the description heretofore.

Interlooping the spacer threads with the textile ground structures ensures that the thread structures resulting therefrom after severing the spacer or connecting threads can no longer be pulled out of the textile ground structures under tensile load, since the interloops would pull tight in such case. This preserves the structural integrity of the medical products obtainable by following the process described in the context of the third aspect of the present invention, at least during the initial postoperative healing phase in the case of partially or completely absorbable products.

Further advantages are that the position and/or length of the spacer threads are freely settable. Better setting is also possible of the angles with which the thread structures resulting (after severing) from the spacer or connecting threads can protrude from the textile ground structures. Furthermore, the textile ground structures may further be configured differently. Moreover, interloop/pore size adjustability is better. Finally, the use of adhesive in regions of the textile ground structures or of threads can also be avoided.

With regard to further features and advantages of the process, especially of the two textile ground structures and/or the spacer threads, the description heretofore is referenced in full.

Further, it is disclosed a process for producing a medical product comprising the steps of:
a) producing, preferably by formed-loop knitting, a textile fabric having a textile ground structure and thread loops which preferably protrude from the textile ground structure, and
b) severing, preferably by cutting through, the thread loops to form thread structures which protrude out of the textile ground structure.

The thread loops are preferably produced using a pile guide bar. Alternatively, however, the thread loops can also be produced via alternating thread insertions using needles and/or pins.

Step a) may be carried out using a formed-loop knitting machine and more particularly a warp-knitting machine.
For this the formed-loop knitting machine preferably has a formed-loop knitting needle bed and a pile guide bar.

The thread loops can be severed, preferably cut through, mechanically, thermally, for example with a hot wire, using ultrasound or using a laser.

With regard to further features and advantages of the process, particularly in respect of the textile fabric and/or the thread loops, the description hereinabove is referenced in full.

Further, it is disclosed a process for producing a medical product in the form of a spacer fabric wherein spacer threads (connecting threads) are interlooped with at least one of two textile ground structures which form opposite each other, or with at least one of two ready-produced textile ground structures opposite each other. The spacer threads may be interlooped with two textile ground structures forming opposite each other or with two ready-formed textile ground structures opposite each other.

The spacer threads are preferably tied into the two textile ground structures in the form of at least one interloop.

The spacer fabric is preferably produced using a formed-loop knitting machine, especially a three-dimensional formed-loop knitting machine, preferably with two formed-loop knitting needle beds.

With regard to further features and advantages of the process, especially in respect of the textile ground structures and/or the spacer or connecting threads, the description hereinabove is referenced in full.

Further features and advantages of the invention will become apparent from the following examples, figure descriptions, figures and also dependent claims. Individual features herein can each be actualized on their own or in combination with each or one another. The embodiments described hereinbelow merely serve to assist understanding of the invention and must not be construed as in any way limiting.

The figures are schematic and show in the case of
- Figures 1 a to e:: embodiments of an invention product in the form of a textile fabric,
- Figures 2a to f:: embodiments of an intermediate product in the form of a spacer fabric not yet comprising free ends of a thread structure protruding from the textile ground structure,
- Figures 3a to d:: an embodiment of an invention product in the form of two textile fabrics laid on top of each other,
- Figure 4:: the lapping diagram for production example 1,
- Figure 5:: the lapping diagram for production example 2,
- Figure 6:: the lapping diagram for production example 3.

While all these production examples 1, 2, 3 have at least some of the thread structures tied pairwise into the textile ground structure in the form of at least one interloop, these production examples do not comprise at least one interloop configured as pillar, tricot, atlas, cord, filet and/or velvet stitch and - though illustrative - are not within the scope of the invention defined by the independent claims 1 or 12.

### Production example 1

The medical product was produced in two stages proceeding from polypropylene threads in that, in a first step, a three-dimensional formed-loop knitting machine was used to produce a three-dimensional formed-loop knit having two opposite loop-formingly knitted, mesh-shaped ground structures and spacer threads which space the ground structures apart from each other and, in a second step, the spacer threads were severed. Severing the spacer threads took place immediately following interloop formation at/in the formed-loop knitting machine and the two severed loop-formingly knitted ground structures were wound up separately.

The three-dimensional formed-loop knit was produced using a three-dimensional formed-loop knitting machine having two formed-loop knitting needle beds (front and back needle beds) and three guide bars (guide bars 7, 4 and 2).
One of the two textile ground structures was produced in atlas lappping (two courses and closed) using guide bar 7 (only one guide bar on the front bed/full threading):
(2-3/2-2/1-2/1-1/1-0/1-1/2-1/2-2) x 2

The second textile ground structure was produced in atlas lapping (two courses and closed) using guide bar 2 (only one guide bar on the back bed/full threading):
(2-2/1-2/1-1/1-0/1-1/2-1/2-2/2-3) x 2

Guide bar 4 was used to connect the two textile ground structures via spacer threads by binding the spacer threads into every second interloop of the ground structures (being formed). The interloops were produced on the front and back needle beds (threaded one in/one out):
(0-1/0-0/0-0/1-0/1-0/0-0/0-0/0-1/0-1/0-0/0-0/1-0/1-0/0-0/0-0/0-1//

Guide bars 7 and 4 were used for the front of the three-dimensional formed-loop knit and guide bars 2 and 4 for the back.

The spacer threads were severed using a hot wire to obtain two medical products each having a textile ground structure and thread structures protruding from the textile ground structure. The medical products had altogether two thread plies, one of which formed the textile ground structure and the other, the thread structures protruding from the textile ground structure.

### Production example 2

The medical product was produced in two stages proceeding from polypropylene threads in that, in a first step, a three-dimensional formed-loop knitting machine was used to produce a three-dimensional formed-loop knit having two opposite loop-formingly knitted, mesh-shaped ground structures and spacer threads which space the ground structures apart from each other and, in a second step, the spacer threads were severed.

Severing the spacer threads took place immediately following interloop formation at/in the formed-loop knitting machine and the two severed loop-formingly knitted ground structures were wound up separately.

The three-dimensional formed-loop knit was produced using a three-dimensional formed-loop knitting machine having two formed-loop knitting needle beds (front and back needle beds) and five guide bars (guide bar 7, guide bar 6, guide bar 4, guide bar 3 and guide bar 2).

The first textile ground structure was produced using two guide bars, namely guide bar 7 and guide bar 6, on the front needle bed (threaded 1 in/1 out):
Guide bar 7:
   (0-1/1-1/2-1/1-1/0-1/1-1/1-2/2-2/3-2/2-2/1-2/2-2/3-2/2-2/2-1/0-0)
Guide bar 6:
   (3-2/2-2/1-2/2-2/3-2/2-2/2-1/1-1/0-1/1-1/2-1/1-1/0-1/1-1/1-2/2-2)

The second textile ground structure was produced using two guide bars, namely guide bar 3 and guide bar 2, on the back needle bed (threaded 1 in/1 out):
Guide bar 3:
   (2-2/3-2/2-2/1-2/2-2/3-2/2-2/2-1/1-1/0-1/1-1/2-1/1-1/0-1/1-1/1-2)
Guide bar 2:
   (1-1/0-1/1-1/2-1/1-1/0-1/1-1/1-2/2-2/3-2/2-2/1-2/2-2/3-2/2-2/2-1)

Guide bar 4 was used to connect the two textile ground structures via spacer threads by binding the spacer threads into every second interloop of the ground structures. The interloops were produced on the front and back needle beds (threaded 1 in/1 out)
Guide bar 4:
(0-1/0-0/0-0/1-0/1-0/0-0/0-0/0-1/0-1/0-0/0-0/1-0/1-0/0-0/0-0/0-1)

Guide bars 7, 6 and 4 were used for the front of the three-dimensional formed-loop knit and guide bars 2, 3 and 4 for the back.

The spacer threads were severed to obtain two medical products each in the form of a fabric having a textile ground structure and thread structures protruding from the textile ground structure. The medical products had altogether three thread plies, two of which formed the textile ground structure and the remaining, the thread structures protruding from the textile ground structure.

### Production example 3

The medical product was produced in two stages proceeding from polypropylene threads in that, in a first step, a three-dimensional formed-loop knitting machine was used to produce a three-dimensional formed-loop knit having two opposite loop-formingly knitted, mesh-shaped ground structures and spacer threads which space the ground structures apart from each other and, in a second step, the spacer threads were severed.

Severing the spacer threads took place immediately following interloop formation at/in the formed-loop knitting machine and the two severed loop-formingly knitted ground structures were wound up separately.

The three-dimensional formed-loop knit was produced using a three-dimensional formed-loop knitting machine having two formed-loop knitting needle beds (front and back needle beds) and five guide bars (guide bar 7, guide bar 6, guide bar 4, guide bar 3 and guide bar 2).

The first textile ground structure was produced using two guide bars, namely guide bar 7 and guide bar 6, on the front needle bed (threaded 1 in/1 out):
Guide bar 7:
   (1-0/1-1/1-2/2-2/2-3/2-2/2-1/1-1) x 3
Guide bar 6:
   (2-3/2-2/2-1/1-1/1-0/1-1/1-2/2-2) x 3

The second textile ground structure was produced using two guide bars, namely guide bar 3 and guide bar 4, on the back needle bed (threaded 1 in/1 out):
Guide bar 3:
   (2-2/2-3/2-2/2-1/1-1/1-0/1-1/1-2) x 3
Guide bar 2:
   (1-1/1-0/1-1/1-2/2-2/2-3/2-2/2-1) x 3

Guide bars 7, 6 and 4 were used for the front of the three-dimensional formed-loop knit and guide bars 2, 3 and 4 for the back.
Guide bar 4:
(1-0/0-0/1-0) x 8

The spacer threads were severed using a hot wire to obtain two medical products each in the form of a textile fabric having a textile ground structure and thread structures protruding from the textile ground structure. Both medical products had in each case three thread plies, two of which formed the textile ground structure and the remaining, the projecting thread structures.

### Description of figures

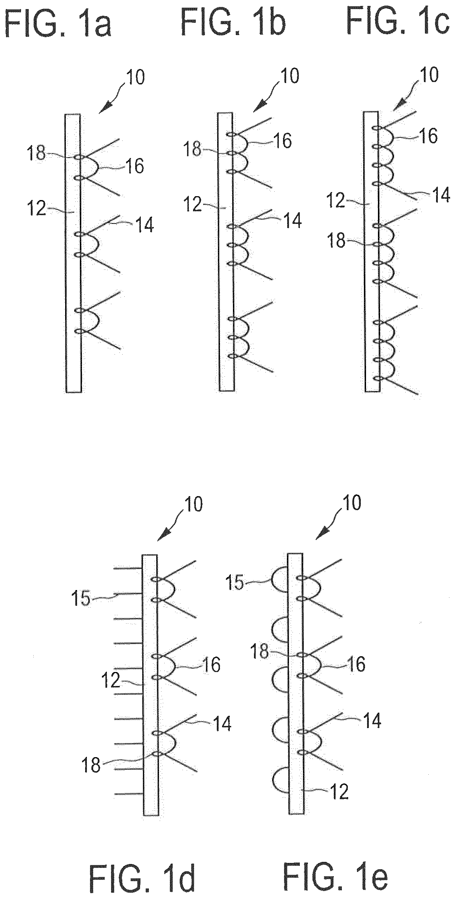

Figures 1a to e show a schematic depiction of a medical product 10 in the form of a textile fabric having a textile ground structure 12 and thread structures 14 protruding from the textile ground structure 12. The thread structures 14 are each tied pairwise into the textile ground structure 12 in the form of an interloop 16 (Figure 1a), two interloops 16 (Figure 1b) or three interloops 16 (Figure 1c). In other words, the thread structures 14 can have two tie-in points 18 (Figure 1a), three tie-in points 18 (Figure 1b) or four tie-in points 18 (Figure 1c) in the textile ground structure 12, or be respectively tied in the textile ground structure 12 two times (Figure 1a), three times (Figure 1b) or four times (Figure 1c).

The thread structures 14 can protrude from one side of the textile ground structure 12 (Figure 1a).

It may alternatively be provided that the medical product 10 has additional thread structures 15 on an opposite side of the textile ground structure 12 which may be configured as cut pile/tuft threads (Figure 1d) or as thread loops (Figure 1e).

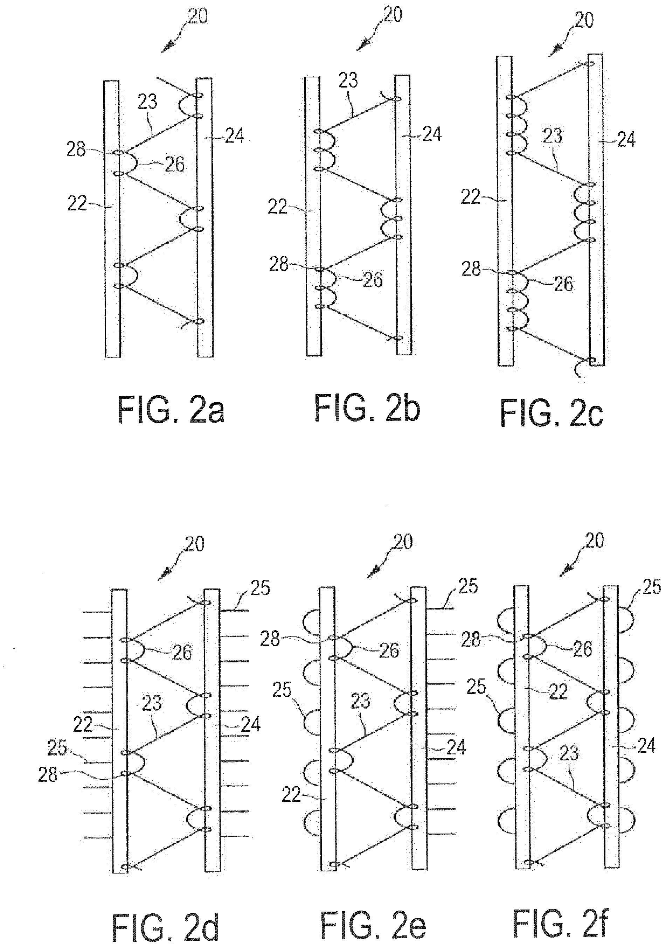

Figures 2a to f show a schematic view of a medical product 20 in the form of a spacer fabric having two textile ground structures 22 and 24 which are mutually spaced apart via spacer threads 23.

The spacer threads 23 may be tied into the textile ground structures 22 and 24 in the form of an interloop 26 (Figure 2a), two interloops 26 (Figure 2b) or three interloops 26 (Figure 2c). In other words, the spacer threads 23 can have two tie-in points 28 (Figure 2a), three tie-in points 28 (Figure 2b) or four tie-in points 28 (Figure 2c) in the textile ground structures 22 and 24, or respectively be tied into the textile ground structure 22 and 24 two times (Figure 2a), three times (Figure 2b) or four times (Figure 2c).

The textile ground structures 22 and 24 may have additional thread structures 25 protruding from the textile ground structures 22 and 24 in the form of cut pile threads and/or thread loops for example on the sides which face away from the spacer threads 23 (Figures 2d to f).
Severing the spacer threads 23, for example with a hot wire or laser, may provide the medical products 10 schematically depicted in Figures 1a to 1f.

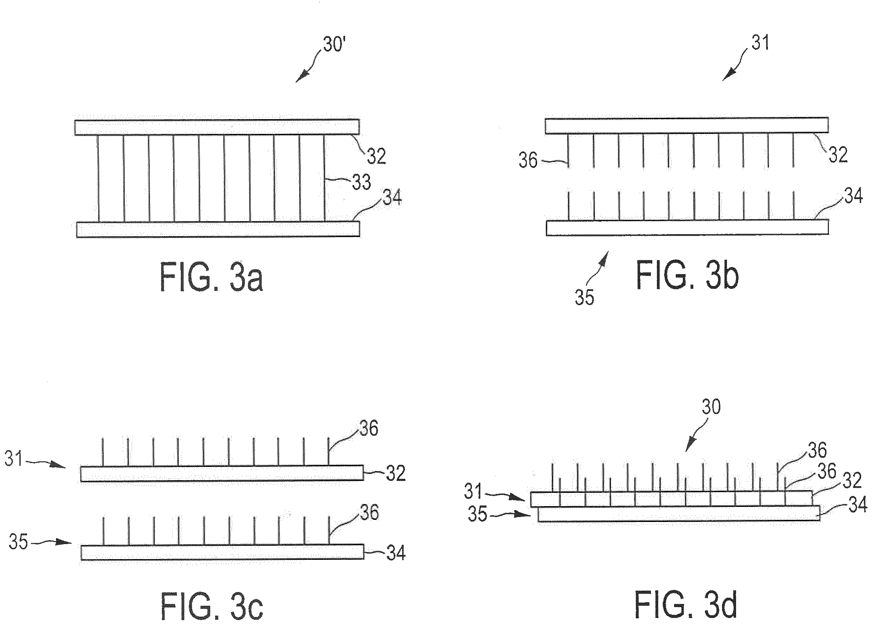

Figures 3a to d are a schematic depiction of the production of a medical product 30 (Figure 1d) proceeding from a spacer fabric 30' having two opposite textile ground structures 32 and 34 which are mutually spaced apart via spacer threads 33 (Figure 1a).

The spacer threads 33 of the spacer fabric 30' are both-sidedly interlooped with the textile ground structures 32 and 34. In this respect, express reference is made to the description hereinabove, especially to the preceding descriptions of figures.

Initially, the spacer threads 33 are cut through to obtain two textile fabrics 31 and 35. The two textile fabrics 31 and 35 each have a textile ground structure 32 or, respectively, 34 and also thread structures 36 sticking out therefrom. The thread structures 36 are thus cut-off or - through spacer threads 33. The thread structures 36 protruding from the textile ground structures 32 and 34 are each one-sidedly interlooped with the textile ground structures 32 and 34.

Next, the two textile fabrics 31 and 35 are laid on top of each other such that the thread structures 36 of the bottom textile fabric 35 protrude through interloops, especially pores, of the top textile fabric 31.

To secure the two textile fabrics 31 and 35 to each other, the thread structures 36 of the bottom textile fabric 35 or of its ground structure 34 may be connected, especially adhered, thermofixed and/or fused, especially ultrafused, to the ground structure 32 of the top textile fabric 31 for example.

This particular form of connecting the two textile fabrics 31 and 35 together significantly raises, especially doubles, the numbers of protruding thread structures 36 on one side of the product 30. This improves to particular advantage the retaining, especially self-retaining, properties of product 30.

The textile fabric 31 is preferably configured to be non-absorbable. The fabric 31 may for example be a polypropylene mesh, in particular a loop-formingly knitted polypropylene mesh. By contrast, the textile fabric 35 is preferably configured to be absorbable. The thread structures 36 are preferably configured to be absorbable.

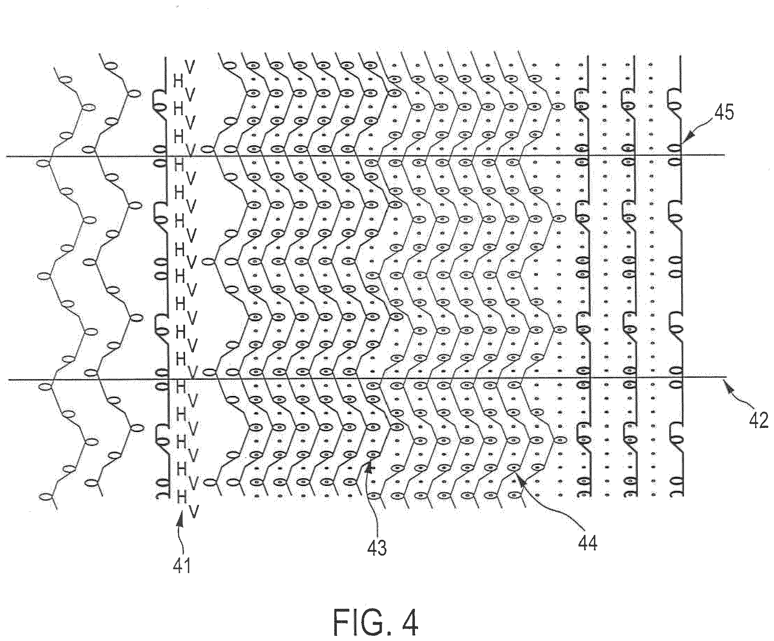

Figure 4 is a schematic showing the lapping diagram 40 for the medical product produced as per Example 1. For clarity, the threads of the individual guide bars are shown side by side and not one above the other.

The reference numerals in Figure 4 have the following meanings:
- Reference numeral 41:: needle bed position (V: front and H: back),
- Reference numeral 42:: repeat line
- Reference numeral 43:: guide bar 7 (ground structure; front needle bed (fully threaded)),
- Reference numeral 44:: guide bar 3 (ground structure; back needle bed (fully threaded)) and
- Reference numeral 45:: guide bar 4 (connecting thread (threaded 1 in, 1 out)).

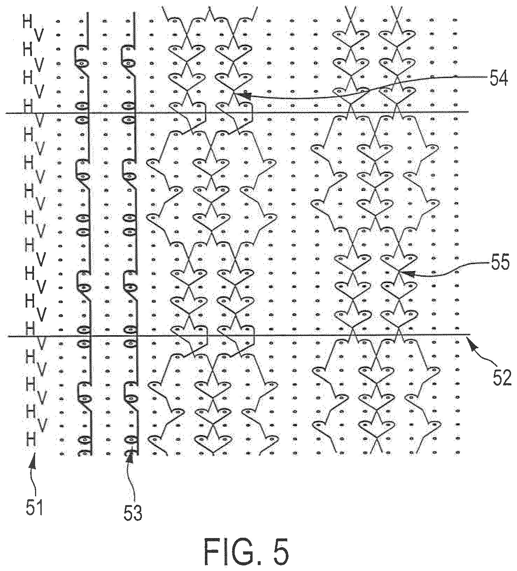

Figure 5 is a schematic showing the lapping diagram 50 for the medical product produced as per Example 2. For clarity, the threads of the individual guide bars are shown side by side and not one above the other.

The reference numerals in Figure 5 have the following meanings:
- Reference numeral 51:: needle bed position (V: front and H: back),
- Reference numeral 52:: repeat line
- Reference numeral 53:: guide bar 4 (connecting thread (1 in, 1 out)),
- Reference numeral 54:: guide bars 3 and 2 (ground structure; produced on back needle bed (threaded 1 in, 1 out)) and
- Reference numeral 55:: guide bars 7 and 6 (ground structure; produced on front needle bed (threaded 1 in, 1 out)).

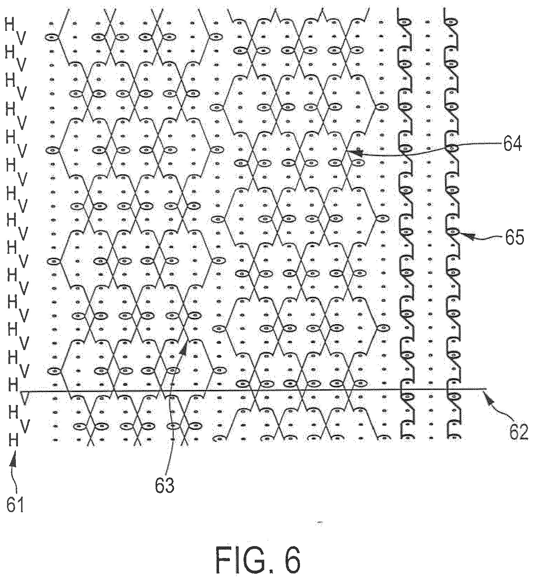

Figure 6 is a schematic showing the lapping diagram 60 for the medical product produced as per Example 3. For clarity, the threads of the individual guide bars are shown side by side and not one above the other.

The reference numerals in Figure 6 have the following meanings:
- Reference numeral 61:: needle bed position (V: front and H: back),
- Reference numeral 62:: repeat line
- Reference numeral 63:: guide bars 7 and 6 (ground structure; produced on front needle bed (threaded 1 in, 1 out)) and
- Reference numeral 64:: guide bars 3 and 2 (ground structure; produced on back needle bed (threaded 1 in, 1 out)) and
- Reference numeral 65:: guide bar 4 (connecting threads (1 in, 1 out).

## Claims

1. Medical product (10, 30), for use in the treatment or management of hernias, prolapses, urinary incontinence and/or dyspareunia in man and/or animal, comprising at least one textile fabric (31, 35) having a textile ground structure (12, 32, 34) and thread structures (14, 36), wherein free ends of the thread structures (14, 36) protrude from the textile ground structure (12, 32, 34) and at least some of the thread structures (14, 36) are tied pairwise into the textile ground structure (12, 32, 34) in the form of at least one interloop (16), **characterised in that** the at least one interloop (16) is configured as pillar, tricot, atlas, cord, filet and/or velvet stitch.

2. Medical product (10, 30) according to Claim 1, **characterized in that** all thread structures (14, 36) are interlooped with the textile ground structure (12, 32, 34).

3. Medical product (10, 30) according to Claim 1 or 2, **characterized in that** the thread structures (14) are tied into the textile ground structure (12) pairwise in the form of a multiplicity of interloops (16).

4. Medical product (10, 30) according to any preceding claim, **characterized in that** the thread structures (14) are formed on one side of the textile ground structure (12) only and thread loops and/or projecting threads in the form of tuft (15) or pile threads are formed on an opposite side of the textile ground structure (12).

5. Medical product (10, 30) according to any preceding claim, **characterized in that** the at least one textile fabric is formed of an absorbable polymer which is selected from the group consisting of polylactide, polyglycolide, poly-ε-caprolactone, poly-para-dioxanone, polytrimethylene carbonate, polyhydroxybutyrate, poly-3-hydroxybutyrate, poly-4-hydroxybutyrate, polyhydroxyvalerate, copolymers thereof, collagen, gelatin, elastin, reticulin, laminin, fibronectin, silk, albumin, fibrin, fibrinogen, starch, amylose, amylopectin, dextran, chitosan, cellulose, alkylcelluloses, especially methylcellulose, carboxyalkylcelluloses, hyaluronic acid, heparin, heparan sulphate, chondroitin sulphate, dermatan sulphate, keratan sulphate, salts thereof and combinations thereof.

6. Medical product (10, 30) according to any of Claims 1 to 4, **characterized in that** the at least one textile fabric is formed of a non-absorbable polymer which is selected from the group consisting of polypropylene, polyethylene, high density polyethylene (HDPE), low density polyethylene (LDPE), high molecular weight polyethylene (HMWPE), ultrahigh molecular weight polyethylene (UHMWPE), polyvinylidene dichloride, polyvinylidene difluoride, polytetrafluoroethylene, polyhexafluoropropylene, polytetrafluoropropylene, polyethylene terephthalate, polypropylene terephthalate, polybutylene terephthalate, polyetheretherketone (PEEK), nylon, nylon-6, nylon-6,6, nylon-6,12, polyurethane, copolymers thereof and combinations thereof.

7. Medical product (10, 30) according to any preceding claim, **characterized in that** the thread structures (14) are absorbable.

8. Medical product (10, 30) according to any preceding claim, **characterized in that** the textile ground structure (12) is configured as interlooped fabric.

9. Medical product (10, 30) according to any preceding claim, **characterized in that** the textile ground structure (12) is configured as formed-loop knit.

10. Medical product (10, 30) according to any preceding claim, **characterized in that** the product (30) includes two textile fabrics (31, 35), each having a textile ground structure (32, 34) and thread structures (36) protruding from the textile ground structure (32, 34).

11. Medical product (30) according to Claim 10, **characterized in that** the two textile fabrics (31, 35) are in a mutually superposed arrangement wherein at least some of the thread structures (36) of the bottom textile fabric (35) project through pores of the top textile fabric (31).

12. Process for producing two medical products (10, 30) according to any preceding claim, wherein spacer threads (33) of a spacer fabric (30') which space apart two mutually opposite textile ground structures (32, 34) from each other are severed to obtain two textile fabrics (31, 35) having a textile ground structure (32, 34) and thread structures (36) having free ends protruding from the textile ground structure (32, 34) wherein the thread structures (36) are tied pairwise into both textile ground structures (32, 34) in the form of at least one interloop (16), **characterised in that** the at least one interloop (16) is configured as pillar, tricot, atlas, cord, filet and/or velvet stitch.

13. Process according to Claim 12, **characterized in that** the spacer threads (33) are interlooped with both textile ground structures (32, 34), to produce the spacer fabric (30').

## Patentansprüche

1. Medizinisches Produkt (10, 30) zur Anwendung bei der Behandlung bzw. Versorgung von Hernien, Prolapsen, Harninkontinenz und/oder Dyspareunia bei Mensch und/oder Tier, umfassend wenigstens ein textiles Flächengebilde (31, 35) mit einer textilen Grundstruktur (12, 32, 34) und Fadenstrukturen (14, 36), wobei freie Enden der Fadenstrukturen (14, 36) aus der textilen Grundstruktur(12, 32, 34) herausragen und mindestens ein Teil der Fadenstrukturen (14, 36) paarweise in die textile Grundstruktur (12, 32, 34) in Form mindestens einer Masche (16) abgebunden sind,
**dadurch gekennzeichnet, dass**
die mindestens eine Masche (16) als Fransen-, Trikot-, Atlas-, Tuch-, Filet- und/oder Samtbindung ausgebildet ist.

2. Medizinisches Produkt (10, 30) nach Anspruch 1, **dadurch gekennzeichnet, dass** alle Fadenstrukturen (14, 36) mit der textilen Grundstruktur (12, 32, 34) vermascht sind.

3. Medizinisches Produkt (10, 30) nach Anspruch 1 oder 2, **dadurch gekennzeichnet, dass** die Fadenstrukturen (14) paarweise, in Form einer Vielzahl von Maschen (16) in die textile Grundstruktur (12) abgebunden sind.

4. Medizinisches Produkt (10, 30) nach einem der vorhergehenden Ansprüche, **dadurch gekennzeichnet, dass** die Fadenstrukturen (14) nur auf einer Seite der textilen Grundstruktur (12) ausgebildet sind und auf einer gegenüberliegenden Seite der textilen Grundstruktur (12) Fadenschlingen und/oder abstehende Fäden in Form von Flor- oder Polfäden (15) ausgebildet sind.

5. Medizinisches Produkt (10, 30) nach einem der vorhergehenden Ansprüche, **dadurch gekennzeichnet, dass** das wenigstens eine textile Flächengebilde aus einem resorbierbaren Polymer gebildet ist, welches ausgewählt ist aus der Gruppe bestehend aus Polylactid, Polyglykolid, Poly-ε-caprolacton, Poly-para-dioxanon, Polytrimethylencarbonat, Polyhydroxybutyrat, Poly-3-Hydroxybutyrat, Poly-4-Hydroxybutyrat, Polyhydroxyvalerat, Copolymere davon, Collagen, Gelatine, Elastin, Retikulin, Laminin, Fibronektin, Seide, Albumin, Fibrin, Fibrinogen, Stärke, Amylose, Amylopektin, Dextran, Chitosan, Cellulose, Alkylcellulosen, insbesondere Methylcellulose, Carboxyalkylcellulosen, Hyaluronsäure, Heparin, Heparansulfat, Chondroitinsulfat, Dermatansulfat, Keratansulfat, Salze davon und Kombinationen davon.

6. Medizinisches Produkt (10, 30) nach einem der Ansprüche 1 bis 4, **dadurch gekennzeichnet, dass** das wenigstens eine textile Flächengebilde aus einem nicht resorbierbaren Polymer gebildet ist, welches ausgewählt ist aus der Gruppe bestehend aus Polypropylen, Polyethylen, Polyethylen hoher Dichte (HDPE), Polyethylen niedriger Dichte (LDPE), hochmolekulares Polyethylen (HMWPE), ultrahochmolekulares Polyethylen (UHMWPE), Polyvinylidendichlorid, Polyvinylidendifluorid, Polytetrafluorethylen, Polyhexafluorpropylen, Polytetrafluorpropylen, Polyethylenterephthalat, Polypropylenterephthalat, Polybutylenterephthalat, Polyetheretherketon (PEEK), Nylon, Nylon 6, Nylon 6-6, Nylon 6-12, Polyurethan, Copolymere davon und Kombinationen davon.

7. Medizinisches Produkt (10, 30) nach einem der vorhergehenden Ansprüche, **dadurch gekennzeichnet, dass** die Fadenstrukturen (14) resorbierbar sind.

8. Medizinisches Produkt (10, 30) nach einem der vorhergehenden Ansprüche, **dadurch gekennzeichnet, dass** die textile Grundstruktur (12) als Maschenware ausgeführt ist.

9. Medizinisches Produkt (10, 30) nach einem der vorhergehenden Ansprüche, **dadurch gekennzeichnet, dass** die textile Grundstruktur (12) als Gewirk ausgebildet ist.

10. Medizinisches Produkt (10, 30) nach einem der vorhergehenden Ansprüche, **dadurch gekennzeichnet, dass** das Produkt (30) zwei textile Flächengebilde (31, 35), mit jeweils einer textilen Grundstruktur (32, 34) und aus der textilen Grundstruktur (32, 34) herausragenden Fadenstrukturen (36), aufweist.

11. Medizinisches Produkt (30) nach Anspruch 10, **dadurch gekennzeichnet, dass** die beiden textilen Flächengebilde (31, 35) übereinander angeordnet sind, wobei wenigstens ein Teil der Fadenstrukturen (36) des unteren textilen Flächengebildes (35) durch Poren des oberen textilen Flächengebildes (31) hindurchragen.

12. Verfahren zur Herstellung von zwei medizinischen Produkten (10, 30) nach einem der vorhergehenden Ansprüche, bei welchem Abstandsfäden (33) eines Abstandstextils (30'), die zwei gegenüberliegende textile Grundstrukturen (32, 34) voneinander beabstanden, durchtrennt werden unter Erhalt von zwei textilen Flächengebilden (31, 35) mit einer textilen Grundstruktur (32, 34) und Fadenstrukturen (36) mit freien Enden, die aus der textilen Grundstruktur (32, 34) herausragen, wobei die Fadenstrukturen (36) paarweise in beide textile Grundstrukturen (32, 34) in Form mindestens einer Masche (16) abgebunden sind,
**dadurch gekennzeichnet, dass**
die mindestens eine Masche (16) als Fransen-, Trikot-, Atlas-, Tuch-, Filet- und/oder Samtbindung ausgebildet ist.

13. Verfahren nach Anspruch 12, **dadurch gekennzeichnet, dass** die Abstandsfäden (33) zur Herstellung des Abstandstextils (30') mit beiden textilen Grundstrukturen (32, 34) vermascht werden.

## Revendications

1. Produit médical (10, 30) pour utilisation dans le traitement ou le soin de hernies, prolapsus, incontinence urinaire et/ou dyspareunie chez homme et/ou animal, comprenant au moins un tissu textile (31, 35) avec une structure de base textile (12, 32, 34) et des structures à fil (14, 36), dans lequel extrémités libres des structures à fil (14, 36) dépassent de la structure de base textile (12, 32, 34) et au moins certains des structures à fil (14, 36) sont liées en paires dans la structure de base textile (12, 32, 34) sous forme d'au moins une maille (16),
**caractérisé en ce que**
ladite au moins une maille (16) est configurée en armure frange, tricot, satin, toile, filet et/ou velours.

2. Produit médical (10, 30) selon la revendication 1, **caractérisé en ce que** tous les structures à fil (14, 36) sont interreliées avec la structure de base textile (12, 32, 34).

3. Produit médical (10, 30) selon la revendication 1 ou 2, **caractérisé en ce que** les structures à fil (14) sont liées en paires dans la structure de base textile (12) sous forme d'une multiplicité de mailles (16).

4. Produit médical (10, 30) selon l'une quelconque des revendications précédentes, **caractérisé en ce que** les structures à fil (14) sont formées seulement sur un côté de la structure de base textile (12) et boucles de fil et/ou fils saillants sous forme de fils de touffe (15) ou fils de poil sont formés sur le côté opposé de la structure de base textile (12).

5. Produit médical (10, 30) selon l'une quelconque des revendications précédentes, **caractérisé en ce que** l'au moins un tissu textile est formé d'un polymère résorbable sélectionné dans le groupe constitué du polylactide, du polyglycolide, du poly-ε-caprolactone, du poly-para-dioxanone, du poly(carbonate de triméthylène), du poly(hydroxybutyrate), du poly(3-hydroxybutyrate), du poly(4-hydroxybutyrate), du poly(hydroxyvalérate), copolymères de ceux-ci, du collagène, de la gélatine, de l'élastine, de la réticuline, de la laminine, de la fibronectine, de la soie, de l'albumine, de la fibrine, du fibrinogène, de l'amidon, de l'amylose, de l'amylopectine, du dextrane, du chitosane, de la cellulose, des alkylcelluloses, en particulier de la méthylcellulose, des carboxyalkylcelluloses, de l'acide hyaluronique, de l'héparine, du sulfate de héparane, du sulfate de chondroïtine, du sulfate de dermatane, du sulfate de kératane, des sels de ceux-ci et des combinaisons de ceux-ci.

6. Produit médical (10, 30) selon l'une quelconque des revendications 1 à 4, **caractérisé en ce que** l'au moins un tissu textile est formé d'un polymère non résorbable sélectionné dans le groupe constitué du polypropylène, du polyéthylène, du polyéthylène à haute densité (HDPE), du polyéthylène à basse densité (LDPE), du polyéthylène à masse moléculaire élevée (HMWPE), du polyéthylène à masse moléculaire plus élevée (UHMWPE), du poly(dichlorure de vinylidène), du poly(difluorure de vinylidène), du polytétrafluoroéthylène, du polyhexafluoropropylène, polytétrafluoropropylène, du poly(téréphtalate d'éthylène), du poly(téréphtalate de propylène), du poly(téréphtalate de butylène), du polyétheréthercétone (PEEK), du nylon, du nylon 6, du nylon 6-6, du nylon 6-12, du polyuréthane, des copolymères de ceux-ci et des combinaisons de ceux-ci.

7. Produit médical (10, 30) selon l'une quelconque des revendications précédentes, **caractérisé en ce que** les structures à fil (14) sont résorbables.

8. Produit médical (10, 30) selon l'une quelconque des revendications précédentes, **caractérisé en ce que** la structure de base textile (12) est configurée sous forme de tissu interrelié.

9. Produit médical (10, 30) selon l'une quelconque des revendications précédentes, **caractérisé en ce que** la structure de base textile (12) est configurée sous forme de tissu à mailles.

10. Produit médical (10, 30) selon l'une quelconque des revendications précédentes, **caractérisé en ce que** le produit (30) comprend deux tissus textiles (31, 35), chacun avec une structure de base textile (32, 34) et des structures à fil (36) dépassant de la structure de base textile (32, 34).

11. Produit médical (30) selon la revendication 10, **caractérisé en ce que** les deux tissus textiles (31, 35) sont superposés l'un sur l'autre, dans lequel au moins une partie des structures à fil (36) du tissu textile (35) inférieure dépassent à travers des pores du tissu textile (31) supérieure.

12. Procédé de production de deux produits médicaux (10, 30) selon l'une quelconque des revendications précédentes, dans lequel des fils d'écartement (33) d'un tissu d'écartement (30'), lesdits fils écartant deux structures de base textile (32, 34) l'une de l'autre, sont coupés en obtenant deux tissus textiles (31, 35) avec une structure de base textile (32, 34) et des extrémités libres de structures à fil (36) dépassant de la structure de base textile (32, 34), dans lequel les structures à fil (36) sont liées en paires dans les deux structures de base textile (32, 34) sous forme d'au moins une maille (16),
**caractérisé en ce que**
ladite au moins une maille (16) est configurée en armure frange, tricot, satin, toile, filet et/ou velours.

13. Procédé selon la revendication 12, **caractérisé en ce que** les fils d'écartement (33) sont interreliés avec les deux structures de base textile (32, 34) pour la production du tissu d'écartement (30').
